# EUROPÄISCHE PATENTSCHRIFT

(11) **EP 2 101 963 B1**
(45) Veröffentlichungstag und Bekanntmachung des Hinweises auf die Patenterteilung: **19.06.2013**
(21) Anmeldenummer: 07818968.5
(22) Anmeldetag: 12.10.2007
(51) Int. Cl.: B25J 9/10, B25J 18/06, B25J 19/00, B25J 9/06, B25J 9/14, A61B 1/00

(54) **WURMFÖRMIGER MECHANISMUS**
WORM-LIKE MECHANISM
MÉCANISME VERMICULAIRE

(30) Priorität: 13.10.2006 DE 102006048531
(43) Veröffentlichungstag der Anmeldung: 23.09.2009
(73) Patentinhaber: Robotics Technology Leaders GmbH, 81249 München (DE)
(72) Erfinder: KNOLL, Alois, 49326 Melle (DE)
(74) Vertreter: Wagner, Bernhard Peter
(86) Internationale Anmeldenummer: PCT/EP2007/008897
(87) Internationale Veröffentlichungsnummer: WO 2008/046566

(56) Entgegenhaltungen:
- EP-A- 0 017 016
- EP-A- 0 249 318
- DE-A1- 4 240 435
- DE-A1- 4 408 730
- DE-A1- 19 833 340
- JP-A- 5 293 787
- US-A- 3 284 964
- US-A- 5 337 732
- US-A- 5 386 741
- US-A- 5 897 488

## Beschreibung

Die Erfindung betrifft einen wurmförmigen Mechanismus, insbesondere einen wurm- oder schlangenartigen verformbaren Handhabungsmechanismus, sowie eine modulartige Betätigungseinheit Für einen solchen Mechanismus. Bekannt sind wurm- oder schlangenartige Mechanismen, beispielsweise für die Endoskopie, die aus einem kontinuierlich verformbaren Träger bestehen. Auf diesem Träger sind senkrecht in bestimmten Abständen Scheiben mit Befestigungspunkten vorgesehen, die mit den Enden von Drahtseilen verbunden sind, welche am Träger entlanggeführt werden. Wird eines oder mehrere dieser Drahtseile gespannt, so führt dies zu einer Krümmung des Mechanismus.

Aus der DE 198 33 340 A1 ist ferner ein wurmförmiger Arbeitsmechanismus bekannt, der als zentrales Trägerelement eine zugelastische Rückstelleinrichtung aufweist, auf der in bestimmten Abständen rechtwinklig scheibenförmige Stützelemente angebracht sind. Um die zentrale zugelastische Rückstelleinrichtung sind drei Aktoren vorgesehen, die jeweils aus einer Reihe aneinander liegender Taschen oder Kissen bestehen. Die einzelnen Taschen oder Kissen liegen dabei jeweils zwischen zwei Stützelementen. Jedem dieser Aktoren kann über einen eigenen Zugangskanal ein flüssiges oder gasförmiges Druckmedium zugeführt werden, so dass die Kissen der Aktoren je nach Füllzustand unterschiedliche Volumina einnehmen. Die Kissen drücken sich gegenseitig auseinander. Werden die drei Aktoren gleichmäßig mit Druckmedium beaufschlagt, so erfolgt eine Längenänderung des Antriebsmechanismus. Werden die drei Aktoren jedoch unterschiedlich beaufschlagt, so ergibt sich eine entsprechende Krümmung des Arbeitsmechanismus. Die Kissen sind hierbei einzeln öder in Gruppen jeweils mit einer eigenen Zuführung für das Druckmedium ausgerüstet.

Aus der US 3,284,964 ist eine schlangenartige, flexible Greifarmstruktur bekannt, die drei Abschnitte mit jeweils einer Vielzahl von Betätigungseinheiten aufweist. Die Zuführung eines Arbeitsmediums zu einer Betätigungseinheit oder Gruppe von Betätigungseinheiten, die jeweils aus zwischen Stützelementen angeordneten Kissen oder Bälgen bestehen, erfolgt ausgehend aus einer zentralen Einheit, an der die Steuerung des Arbeitsdrucks für jede Betätigungseinheit oder Gruppe von Betätigungseinheiten vorgenommen wird. Diese zentrale Einheit befindet sich außerhalb der flexiblen Greifarmstruktur und ist normalerweise groß und schwer. Vor allem aber ergeben sich aus dieser Bauweise große Leitungslängen, so dass es in Folge der daraus resultierenden Totzeiten zu einer schlechten Steuer- oder Regelbarkeit des Mechanismus kommt. Außerdem weist die Basis des Greifarms, durch die sämtliche Leitungen geführt werden, einen großen Umfang auf, der mit der Länge des Greifarms und/oder der Anzahl der Segmente wächst. Dies führt dazu, dass derartige Mechanismen normalerweise sehr dick im Verhältnis zu ihrer Länge werden, wodurch ihre Beweglichkeit deutlich eingeschränkt ist.

Anstelle von Druckkissen oder Bälgen, die sich bei Druckbeaufschlagung ausdehnen und eine Verlängerung bewirken, können auch so genannte künstliche Muskeln eingesetzt werden, wie sie die Firma Festo AG & Co. KG, Esslingen. Deutschland, anbietet.

Die JP 05-293787 betrifft einen wurmförmigen Mechanismus, bei dem die einzelnen Zellen der Stellelemente über Drosselöffnungen mit einer Druckleitung verbunden sind, sodass ihnen ständig ein Druckmedium zugeführt wird. Um unterschiedliche Ausdehnungszustände der einzelnen Zellen zu realisieren, kann über Ventile Druck aus den Zellen abgelassen werden.

Die US 5,337,732 A zeigt einen wurmförmigen Mechanismus, dessen einzelne Betätigungseinheiten über Ballone verfügen, mit denen sich die Betätigungseinheiten in einem Körperhohlraum an dessen Wänden anlegen können, um sich dann raupenartig vorwärts zu bewegen. Die hierfür erforderliche Längenveränderung, also ein Zusammenziehen und Ausdehnen, wird dabei von einer Kolbenzylindereinheit realisiert, die je nach Druckbeaufschlagung eine Verlängerung oder eine Verkürzung des Abstands zur benachbarten Betätigungseinheit bewirkt.

Ferner können auch Betätigungseinheiten vorgesehen sein, die mit vier um die Längsachse der Betätigungseinheit herum verteilt angeordneten Säcken versehen sind, von denen jeder über ein erstes Ventil mit Druck beaufschlagbar und über ein zweites Ventil entlüftbar ist, um ein Biegen des wurmförmigen Mechanismus zu bewirken.

Die EP 0 017 016 A1 betrifft einen flexiblen Roboterarm, der zwei oder mehr Abschnitte aufweist, die jeweils aus einer Vielzahl von Kissen oder Elementen bestehen, die über entsprechende konvexe Flächen so miteinander in Kontakt gehalten werden, dass eine Verbiegung des wirbelsäulenartigen Aufbaus möglich ist. Hierzu sind beispielsweise Kolbenzylinderanordnungen zwischen Flanschen benachbarter Elemente angeordnet, um diese auf der außen liegenden Seite einer Biegung auseinanderzudrücken. Dabei wird Jedem Zylinder von einer Zuführleitung über einen Einlass/Auslass Druckluft zugeführt. Auf der innen liegenden Seite der Biegung wird in entsprechender Weise die in den Zylindern vorhandene Luft über den Auslass/Einlass und die Zuführleitung, die Jetzt als Abführleitung dient, herausgedrückt.

Ferner können die Elemente auch aufgeteilt sein, und zwei einander gegenüberliegende Flanschelemente besitzen, zwischen denen Servomotoren, die beispielsweise als hydraulische oder pneumatische Bälge ausgebildet sind, vorgesehen sind. Die Druckbeaufschlagung und Druckentlastung erfolgt wiederum über entsprechende Zuführ- bzw. Abführleitungen. Dabei werden sämtliche Elemente eines Abschnitts gleichzeitig in gleicher Weise betätigt.

Die DE 44 08 730 A1 beschreibt einen steuerbaren Instrumentenkanal, bei dem die Aktuatoren im Kanalmantel eingebettet sind. Diese Aktuatoren können beispielsweise Streifen aus Formgedächtnis- oder Thermobimetall sein. Ferner können auch Dehnungsmessstreifen, Schalter oder Drucksensoren in oder an dem Kanalmantel positioniert sein.

Die US 5,897,488 A betrifft ein Endoskop oder Katheter mit einem Biegeabschnitt mit einer Vielzahl von Gelenkstücken. Um den Biegeabschnitt aus Gelenkstücken in der einen oder anderen Richtung verbiegen zu können, sind Zugdrähte vorgesehen, die von künstlichen Muskeln betätigt werden.

Hier ist also nur gezeigt, dass zur Betätigung von Zugdrähten künstliche Muskeln eingesetzt werden können.

Die EP 0 249 318 A1 beschreibt Betätigungselemente für einen Roboter oder Manipulatorarm, bei dem Stäbe oder Stangen aus Federstahl oder dergleichen einen Blegeabschnitt bilden. Die Stäbe oder Stangen sind mit ihren einen Enden mit Stellelementen verbunden, während ihre anderen Enden an einer Stützplatte gehalten sind. Mit Hilfe der Stellelemente können die Stäbe oder Stangen verdreht werden, um eine Verdrehung der Stützplatte gegenüber dem Basisabschnitt zu realisieren. Um Biegungen des Biegeabschnitts zu erhalten, können die Stangen mit Hilfe von Kolbenzylindereinheiten, die in beiden Richtungen betätigt werden können, in ihrer Längsrichtung verschoben werden.

Der Erfindung liegt die Aufgabe zugrunde, einen weiteren wurmförmigen Mechanismus bereitzustellen, der insbesondere bei vereinfachtem schlankem Aufbau eine präzise Steuerung oder Regelung seiner Bewegungen ermöglicht.

Diese Aufgabe wird durch den wurmförmigen Mechanismus nach Anspruch 1 bzw. nach Anspruch 2, sowie durch die modulartige Betätigungseinheit nach Anspruch 19 gelöst. Vorteilhafte Ausgestaltungen und Weiterbildungen der Erfindung sind in den Unteransprüchen beschrieben.

Erfindungsgemäß ist also bei einem wurmförmigen Mechanismus mit einer sich in Längsrichtung des Mechanismus erstreckenden Trägerstruktur und mit zumindest zwei in Längsrichtung der Trägerstruktur hintereinander angeordneten Betätigungseinheiten, von denen jede zwei Stützelemente und zumindest ein zwischen den Stützelementen angeordnetes Betätigungselement aufweist, durch das die Stützelemente relativ zu einander bewegbar sind, vorgesehen, dass die Betätigungselemente einzeln über eine zentrale Leitung ansteuerbar sind. Dabei sind die Betätigungselemente hydraulische oder pneumatische, zumindest auf Zug arbeitende Betätigungselemente, die jeweils über ein Ventil an die zentrale Leitung angeschlossen, die eine Zuführleitung zum Zuführen eines hydraulischen oder pneumatischen Arbeitsmediums zu den Betätigungselementen ist.

Die zentrale Ansteuerung der Betätigungselemente in den Betätigungseinheiten zur Verformung des Mechanismus über eine zentrale Leitung, die sich entlang des wurmförmigen Mechanismus erstreckt, ermöglicht kurze Schaltzeiten, die auch für eine individuelle Nachregelung der durch die einzelnen Betätigungseinheiten erzielten Bewegungen oder Verformungen geeignet ist. Dabei ermöglicht es die Verwendung hydraulischer oder pneumatischer Betätigungselemente, denen ein Arbeitsmedium über Ventile von einer zentralen Leitung zugeführt wird, die als Rohr oder Schlauch ausgebildet sein kann, das unter Druck steht, dass das Arbeits- oder Druckmedium ohne wesentliche Totzeiten über die an die zentrale Zuführleitung angeschlossenen Ventile den einzelnen Betätigungselementen schnell zugeführt werden kann. Da die Ventile nahe den Betätigungselementen angeordnet sind, ergeben sich extrem kurze Zuführwege zu den Betätigungselementen wodurch die effektiven Schaltzeiten drastisch verkürzt werden können.

Zweckmäßigerweise ist dabei vorgesehen, dass die Ventile jeweils eine erste offene Stellung zum Verbinden des oder der Betätigungselemente mit der Zuführleitung, eine Schließstellung und eine zweite offene Stellung zum Auslassen des Arbeitsmediums aus dem oder den Betätigungselementen aufweist.

Grundsätzlich ist es denkbar, dass die Ventile zum Entlasten der pneumatischen oder hydraulischen Betätigungselemente in ihrer zweiten Offenstellung die Betätigungselemente mit der Umgebung verbinden, sofern als Arbeitsmedium ein Gas oder eine Flüssigkeit eingesetzt wird, die ohne Gefahr für die Umwelt oder die Arbeitsumgebung, in der der Mechanismus eingesetzt wird, in die Umgebung abgelassen werden kann. Zweckmäßigerweise ist jedoch vorgesehen, dass die Ventile in der zweiten offenen Stellung die Betätigungselemente mit einer Rücklaufleitung der mit einer Auslassleitung verbinden. Hierdurch lässt sich entweder eine Rückgewinnung des Arbeitsmediums oder eine Ableitung des Arbeitsmediums in einen Bereich erzielen, in dem die Arbeitsumgebung des Mechanismus nicht beeinträchtigt wird, was für eine Vielzahl von Anwendungszwecken, wie beispielsweise bei der Endoskopie erforderlich und in fast allen Anwendungen zweckmäßig ist, da der Arbeits- oder Einsatzbereich des wurmförmigen Mechanismus nicht beeinträchtigt wird.

Die Rücklaufleitung kann auch als Sauf- oder Unterdruckleitung ausgelegt und mit einer entsprechenden Vakuumquelle verbunden sein, um den Druckabbau in den Betätigungselementen zu beschleunigen.

Bei einer vorteilhaften Weiterbildung der Erfindung ist vorgesehen, dass die Ventile mittels Piezoelementen, insbesondere mittels einer Konstruktion gestapelter Piezoelemente schaltbar sind. Hierdurch lässt sich eine schnelle und zuverlässige Schaltbarkeit der Ventile erreichen.

Aufgrund der Verwendung derartiger, sehr schnell steuerbarer Ventile und der kurzen oder sogar verschwindenden Leitungen zwischen Ventilausgang und Betätigungselement sind extrem hohe Frequenzen bei der Steuerung der Ventile möglich, so dass sich beispielsweise beim Ablassen von Druck Nachgiebigkeiten der Betätigungselemente einstellen lassen, dass also der Druck der Betätigungselemente zur passiven Längenänderung definiert langsam abgebaut wird, so dass sich Gegenmomente des Mechanismus fein einstellen und regeln lassen.

Gemäß einer zweckmäßigen Weiterbildung der Erfindung ist vorgesehen, dass jede Betätigungseinheit eine Treiberschaltung aufweist, die mit einer zentralen Steuerschaltung verbunden ist und die Schaltsignale für die Ventile der Betätigungseinheit in Abhängigkeit von von der zentralen Steuerschaltung empfangenen Steuerinformation liefert, wobei die Treiberschaltung einen Steuerinformationsverarbeitungskreis umfasst, der Steuersignale für die die Ventile schaltende Ventiltreiberkreise in Abhängigkeit von der empfangenen Steuerinformation erzeugt.

Um die jeweils erreichten Bewegungen und/oder Verformungen für eine Regelung zu erfassen, ist es erfindungsgemäß vorgesehen, dass jede Betätigungseinheit einen oder mehrere Bewegungsfühler aufweist, deren Ausgangssignale von der Treiberschaltung erfasst werden.

Wird die Stellinformation für jedes Betätigungselement dabei zentral ermittelt, so ist es zweckmäßig, wenn die Treiberschaltung einen Übertragungskreis für die Übertragung der Ausgangssignale von dem oder den Bewegungsfühlern an die zentrale Steuerschaltung aufweist.

Es ist aber auch möglich, dass die Treiberschaltung einen Reglerkreis aufweist, dem die von der zentralen Steuerschaltung empfangene Steuerinformation und die Ausgangssignale von dem oder den Bewegungsfühlern als Regelinformation zugeführt sind und der Steuersignale für die die Ventile schaltende Ventiltreiberkreise in Abhängigkeit von der Regelinformation liefert.

Als Bewegungsfühler für Knick-. Längs- oder Drehbewegungen können zweckmäßigerweise ein oder mehrere Dehnungsmessstreifen vorgesehen sein, die auf der Trägerstruktur angebracht sind. Daneben ist es möglich, zwischen den Stützelementen der Betätigungseinheit ein oder mehrere Tauchspulen so anzuordnen, dass sie die Relativbewegung der Stützelemente erfassen. Bei einer anderen Ausgestaltung der Erfindung ist vorgesehen, dass als Bewegungsfühler ein optischer Lagedetektor mit einer Lichtquelle und einem positionsempfindlichen Photoempfänger vorgesehen ist, die jeweils an gegeneinander bewegbaren Elementen der Betätigungseinheit angeordnet sind, wobei der Lagedetektor ein der relativen Bewegung zwischen diesen Elementen entsprechendes Ausgangssignal liefert.

Als Betätigungselemente können Druckzylinder oder auch sich unter Druck zusammenziehende pneumatische oder hydraulische Arbeitselemente, insbesondere so genannte künstliche Muskeln vorgesehen sein. Dabei ist es auch möglich, die verschiedenen längenveränderlichen Betätigungselemente Je nach den gewünschten Bewegungsabläufen und der Steuerbarkeit bzw. Regelbarkeit miteinander zu kombinieren.

Dabei ist es auch möglich, dass die Betätigungselemente bei Druckentlastung eine Rückstellwirkung liefern. Ferner ist es bei verschiedenen Anwendungen zweckmäßig, dass den Betätigungselementen ein oder mehrere Rückstellelemente zugeordnet sind.

Werden Jedem Betätigungselement einer Betätigungseinheit ein oder mehrere entgegengesetzt wirkende Betätigungselemente zugeordnet, so können die Betätigungselemente dann die Rolle von Muskeln übernehmen, die wie Protagonist und Antagonist zusammenarbeiten, um eine definierte Bewegung sehr präzise ausführen zu können.

Bei einer zweckmäßigen Weiterbildung der Erfindung ist vorgesehen, dass das oder die Betätigungselemente in der Betätigungseinheit so angeordnet sind, dass sie bei Aktivierung eine Längenänderung bewirken.

Für die meisten Anwendungsfälle ist es zweckmäßig, dass in einer Betätigungseinheit zwei, drei oder mehr Betätigungselemente so angeordnet sind, dass sie bei entsprechender Aktivierung eine definierte Verkippung der beiden Stützelemente gegeneinander in einer definierten radialen Richtung bewirken.

Gemäß einer anderen Ausgestaltung der Erfindung ist vorgesehen, dass die Betätigungselemente so in einer Betätigungseinheit angeordnet sind, dass sie bei Aktivierung eine Verdrehung der beiden Stützelemente um die Längsrichtung der Trägerstruktur bewirken.

Je nach Anwendungsfall können in einem wurmartigen Mechanismus gemäß der vorliegenden Erfindung verschiedene Betätigungseinheiten miteinander kombiniert werden, die jeweils nur eine Längenveränderung oder eine Verbiegung oder Verkippung oder eine Verdrehung um die Achse des wurmförmigen Mechanismus bewirken. Es ist aber auch möglich, die verschiedenen Bewegungen in einer Betätigungseinheit zu kombinieren, wobei der zentrale Träger und/oder die Lagerung der Stützelemente so ausgebildet sein muss, dass die Stützelemente der Betätigungseinheit den gewünschten Bewegungen und Verformungen folgen können.

Zweckmäßigerweise sind die Stützelemente als Stützscheiben ausgebildet, die einen Durchgang für die zentrale Leitung aufweisen. Der Durchgang für die zentrale Leitung muss dabei nicht im Bereich der Mittelachse des Mechanismus liegen, sondern kann auch seitlich dazu versetzt sein. Die zentrale Leitung ist dabei jeweils so auszulegen, dass sie den Bewegungen der einzelnen Betätigungseinheiten ohne weiteres folgen kann ohne diese zu stören.

Bei einer vorteilhaften Ausgestaltung der Erfindung ist vorgesehen, dass die Trägerstruktur einen kontinuierlich verformbaren rohr- oder schlauchförmigen Träger aufweist, an dem die Stützelement in definierten axialen Abständen befestigt sind. Die Verwendung eines rohr-oder schlauchförmigen Trägers als Trägerstruktur des Mechanismus ermöglicht es, die verschiedenen Zuführ- und Abführleitungen sowie die elektrischen Steuerleitungen zusätzlich geschützt durch den wurmförmigen Mechanismus zu führen. Es ist aber auch denkbar, den rohr- oder schlauchförmigen Träger selbst als Zuführleitung für das Arbeitsmedium einzusetzen, wodurch sich eine weitere Vereinfachung des Aufbaus ergibt.

Bei einer anderen Ausgestaltung der Erfindung ist vorgesehen, dass die Trägerstruktur aus einer Mehrzahl von gleichartigen Trägersegmenten besteht, die modulartig miteinander verbunden sind.

Durch die Verwendung einer wirbelsäulenähnlichen Trägerstruktur aus gleichartigen Trägersegmenten lässt sich die Herstellung verschiedenartiger, insbesondere verschieden langer wurmförmiger Mechanismen wesentlich rationalisieren, wodurch Herstellungskosten gesenkt werden können. Die modulartigen Trägersegmente können dabei auch aus rohr- oder schlauchförmigen Trägern mit daran angeordneten Stützelementen bestehen, wobei die rohr- oder schlauchförmigen Trägerabschnitte an ihren Enden mit entsprechenden Kupplungsgliedern ausgerüstet sein müssen, so dass der daraus aufgebaute, fertige, wurmartige Mechanismus als Trägerstruktur einen durchgehenden, verformbaren Träger aufweist, der aus einzelnen Abschnitten zusammengesetzt ist.

Bei einer anderen Ausgestaltung der Erfindung ist vorgesehen, dass jede der Betätigungseinheiten ein Trägersegment aufweist, das eine erste Stützplatte, die fest mit einem Ende eines Längsträgers verbunden ist, und eine zweite Stützplatte umfasst, die über eine Gelenkverbindung schwenkbar und/oder drehbar an dem anderen Ende des Längsträgers gehalten ist, wobei die erste Stützplatte auf ihrer vom Längsträger abgewandten Seite Befestigungsmittel zum Befestigen einer zweiten Stützplatte eines benachbarten Trägersegments aufweist.

Gemäß einer anderen Ausführungsform der Erfindung ist es zweckmäßig, wenn jede der Betätigungseinheiten ein Trägersegment umfasst, das eine Stützplatte aufweist, die fest mit einem Ende eines Längsträgers verbunden ist und die auf ihrer vom Längsträger abgewandten Seite ein Gelenklager trägt, wobei der Längsträger an seinem anderen Ende ein mit dem Gelenklager korrespondierendes Gelenkglied trägt, so dass der Längsträger einer Betätigungseinheit schwenkbar und/oder drehbar in dem Gelenklager an der Stützplatte der nächsten Betätigungseinheit lagerbar ist.

Um bei einer bekannten Anwendungslage für den wurmförmigen Mechanismus eine Vorspannung in einer Vorzugsrichtung oder eine Entlastung gegenüber statisch anliegenden Kräften, wie etwa der Schwerkraft zu erzielen, ist es zweckmäßig, wenn in einem oder mehreren der Betätigungseinheiten Zug- und/oder Druckfederelemente vorgesehen sind, die zum Ausgleich von in einer geplanten Anwendungslage des Mechanismus zu erwartenden statischen Kräften diesen intern abstützen. Hierdurch lässt sich erreichen, dass die einzelnen Betätigungselemente nur noch die Differenzkraft für die gewünschte Bewegung oder Verstellung aufzubringen haben.

Die Erfindung wird im Folgenden beispielsweise anhand von in der Zeichnung dargestellten Ausführungsbeispielen näher erläutert. Es zeigen:
Figur 1 eine stark vereinfachte schematische Darstellung eines wurmförmigen Mechanismus gemäß der Erfindung mit einer kontinuierlichen verformbaren Trägerstruktur;
Figur 2 eine stark vereinfachte schematische Darstellung eines wurmförmigen Mechanismus gemäß der Erfindung mit einer verformbaren Trägerstruktur mit wirbelsäulenähnlicher Anordnung der Trägersegmente;
Figur 3 eine vereinfachte schematische, perspektivische Ansicht zweier aneinander anschließender Betätigungseinheiten des wurmförmigen Mechanismus gemäß einem ersten Ausführungsbeispiel der Erfindung;
Figur 4 eine vereinfachte schematische Schnittdärstellung einer Betätigungseinheit eines wurmförmigen Mechanismus gemäß einem anderen Ausführungsbeispiel der Erfindung;
Figuren 5(a) bis (d) stark vereinfachte schematische Anrichten einer Betätigungseinheit eines erfindungsgemäßen Mechanismus zur Veranschaulichung verschiedener Anordnungsmöglichkeiten von Betätigungselementen;
Figur 6 ein vereinfachtes schematisches hydraulisch/pneumatisches Schaltbild zur Veranschaulichung der Druckbeaufschlagung von als Betätigungselementen eingesetzten künstlichen Muskeln;
Figur 7 ein vereinfachtes schematisches Schaltbild entsprechend Figur 6 bei Verwendung von Druckzylindern;
Figur 8 ein vereinfachtes schematisches Schaltbild zur Veranschaulichung der elektrischen Ansteuerung der einzelnen Betätigungseinheiten;
Figur 9 ein vereinfachtes schematisches Blockschaltbild einer Treiberschaltung in den Betätigungseinheiten; und
Figur 10 ein vereinfachtes schematisches Blockschaltbild einer anderen Treiberschaltung der Betätigungseinheiten.

In den verschiedenen Figuren der Zeichnung sind einander entsprechende Bauteile und Schaltungselemente durchgehend mit den gleichen Bezugszeichen versehen.

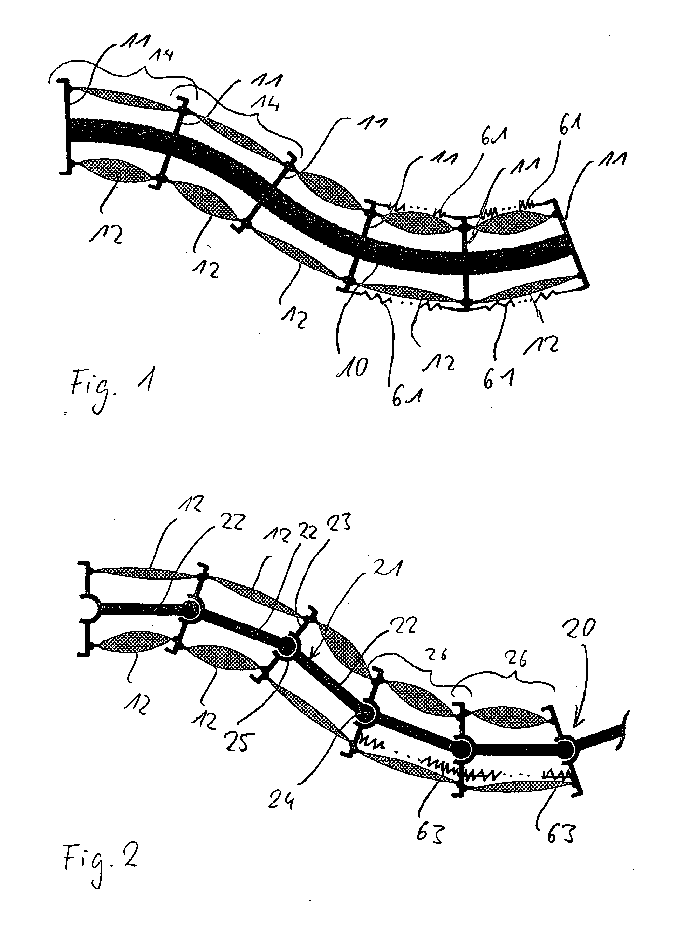

Figur 1 zeigt eine erste Ausgestaltung eines erfindungsgemäßen wurmförmigen Mechanismus mit einer Trägerstruktur 10, die als kontinuierlich verformbarer Träger ausgebildet ist, wie unten näher erläutert wird. An der Trägerstruktur 10 sind Stützelemente 11 angeordnet, die beispielsweise als Platten oder Scheiben, aber auch als Stern- oder kreuzförmig angeordnete Stützstreben ausgebildet sein können.

Zwischen jeweils zwei in Längsrichtung der Trägerstruktur benachbarten Stützelementen 11 sind Betätigungselemente 12 angeordnet, die in Figur 1 als künstliche Muskeln angedeutet sind, die sich bei Druckbeaufschlagung verkürzen.

Die Stützelemente 11, die sich quer zur Längsrichtung der Trägerstruktur 10 erstrecken, sind an dieser in Längsrichtung der Trägerstruktur 10 voneinander beabstandet angebracht. Dabei bilden jeweils zwei benachbarte Stützelemente 11 zusammen mit den dazwischen liegenden Betätigungselementen 12 und dem Abschnitt der Trägerstruktur 10 zwischen den Betätigungselementen 12 eine Betätigungseinheit 14, wobei bei der in Figur 1 dargestellten Ausführungsform der Erfindung die Stützelemente 11 jeweils zu zwei benachbarten Betätigungseinheiten 14 gehören.

Figur 2 zeigt einen anderen verformbaren, wurmförmigen Mechanismus gemäß der vorliegenden Erfindung, bei dem die Trägerstruktur 20 wirbelsäulenähnlich ausgebildet ist und Trägersegmente 21 aufweist, von denen jedes einen Längsträger 22 und eine Stützplatte 23 aufweist. Der Längsträger 22 ist an seinem von der Stützplatte 23 abgewandten Ende als Gelenkglied 24 ausgebildet, das in einem an der Stützplatte 23 und/oder dem Längsträger 22 der benachbarten Betätigungseinheit 26 ausgebildeten Gelenklager 25 aufgenommen ist.

In Figur 2 sind das Gelenkglied 24 und das Gelenklager 25 als Elemente eines Kugelgelenks dargestellt, das sowohl Schwenk- als auch Drehbewegungen ermöglicht. Es ist aber auch denkbar, dass die Gelenkverbindung zwischen zwei Betätigungseinheiten 26 des Mechanismus so ausgebildet sind, dass sie entweder nur eine Drehbewegung um die Längsachse des Mechanismus, oder eine Schwenk- oder Kippbewegung relativ zur Längsachse des Mechanismus mit einem oder zwei Freiheitsgraden zulässt. Die Ausbildung von Gelenkglied und Gelenklager ist dabei je nach den Anwendungserfordernissen und den zu übertragenden Kräften auszulegen.

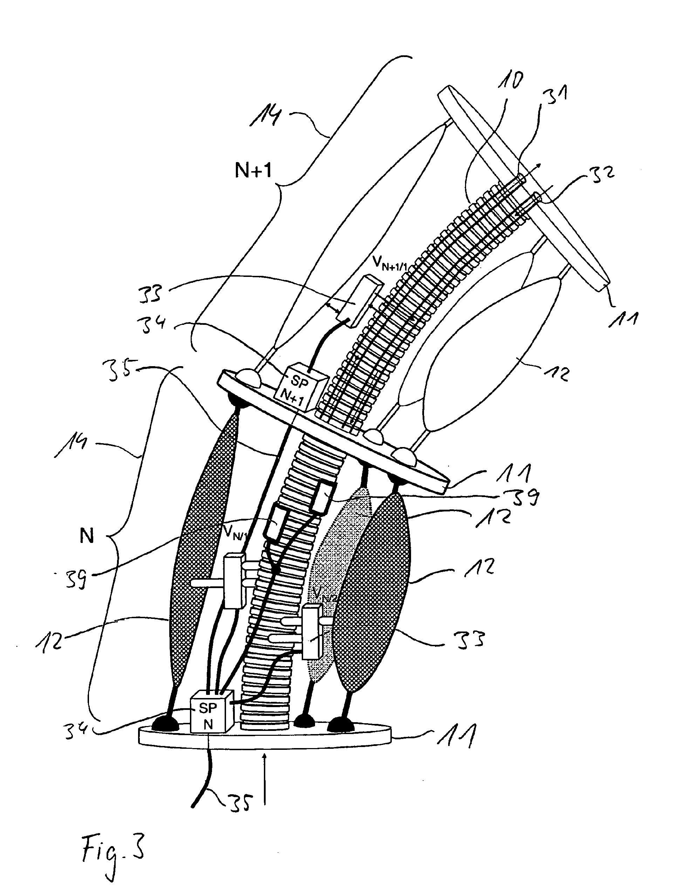

Figur 3 zeigt die N-te und (N+1)-te Betätigungseinheit 14 eines wurmförmigen Mechanismus, der eine kontinuierlich verformbare Trägerstruktur 10 aufweist, wie sie in Figur 1 angedeutet ist. Die kontinuierliche Trägerstruktur 10 wird gemäß Figur 3 beispielsweise von einem Stützschlauch, der aus ringförmigen, ineinander greifenden und gegeneinander verschiebbaren Stützringen besteht, gebildet. Anstelle eines derartigen Stützschlauches, durch den nicht nur eine oder mehrere zentrale Leitungen 31, 32 und gegebenenfalls auch Datenleitungen, sondern auch gemäß einer anderen Ausgestaltung ein Arbeitsmedium für die einzelnen Betätigungselemente 12 unmittelbar geführt sein kann, kann auch ein Wellschlauch oder eine flexible, gitterförmige Schlauch- oder Rohrstruktur vorgesehen sein. Die Trägerstruktur 10 besitzt dabei eine gewisse, je nach Anwendung des Mechanismus anzupassende Steifigkeit.

Die Trägerstruktur 10 lässt sich auch aus Stützschlauchabschnitten aufbauen, die an den Übergängen zwischen den Betätigungseinheiten 14 in geeigneter Weise aneinandergekoppelt sind, um einen modularen Aufbau zu erhalten.

An der Trägerstruktur 10 sind als Stützelemente kreisförmige Stützplatten 11 angeordnet, wobei zwischen den beiden Stützplatten 11 einer Betätigungseinheit 14 im dargestellten Ausführungsbeispiel drei als künstliche Muskeln angedeutete Betätigungselemente 12 angeordnet sind, die in Umfangsrichtung um die Trägerstruktur 10 verteilt positioniert sind. Je nach Einsatzbereich kann die umfangsmäßige Verteilung der Betätigungselemente 12 gleichmäßig oder ungleichmäßig sein. Letzteres insbesondere dann, wenn, wie z.B. bei einer horizontalen Anwendung aufgrund der Schwerkraft die radiale Krafteinwirkung auch im Ruhezustand ungleichmäßig ist.

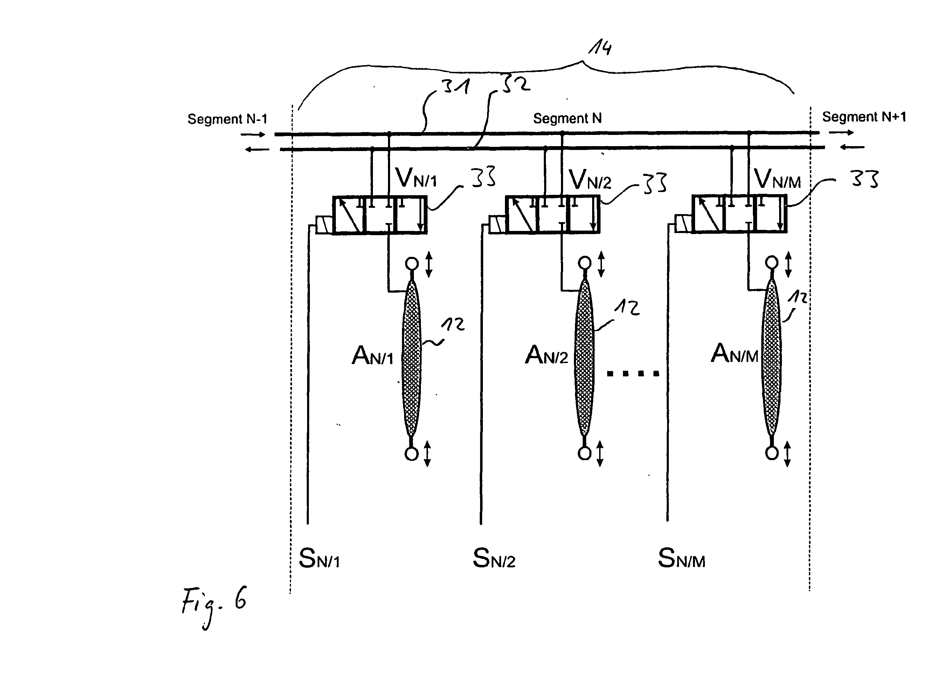

Wie in Figur 3 und 6 dargestellt ist, sind die drei Betätigungselemente 12 über zugeordnete Ventile 33 mit der Zuführleitung 31 zum Zuführen eines hydraulischen oder pneumatischen Arbeitsmediums und mit einer Auslass- oder Rücklaufleitung 32 für das Arbeitsmedium verbindbar.

Wie in Figur 6 gezeigt ist, sind die Ventile 33 als 3/3-Wegeventile ausgebildet, die eine zentrale Schließstellung sowie eine erste offene Stellung, in der die Betätigungselemente 12 mit der Zuführleitung 31 verbunden sind, und eine zweite offene Stellung aufweisen, in der die Betätigungselemente 12 mit der Rücklauf- oder Auslassleitung 32 verbunden sind.

Anstelle der Verbindung der Betätigungselemente 12 mit der Rücklauf- oder Auslassleitung 32 zum Auslassen von Arbeitsmedium können die Ventile 33 auch so ausgelegt sein, dass sie die Betätigungselemente 12 direkt mit der Umgebung verbinden, falls als Arbeitsmedium beispielsweise Luft eingesetzt wird, die ohne Beeinträchtigung der Umwelt bei vielen Anwendungen ohne weiteres ins Freie abgelassen werden kann. In entsprechender Weise ist es denkbar, falls der Mechanismus unter Wasser eingesetzt wird, Wasser als Arbeitsmedium auch einfach in die Umgebung auszulassen. Bei anderen Anwendungsfällen, beispielsweise in der Medizin, ist es jedoch erforderlich, das Arbeitsmedium aus dem wurmförmigen Mechanismus herauszuführen und entweder zur Rückgewinnung in einen Sumpf zurückzuleiten oder an einer Stelle in die Umwelt zu entlassen, wo dies für die Handhabung des wurmförmigen Mechanismus unschädlich ist.

Die Ventile 33 können als Magnetventile ausgebildet sein, die schnell und zuverlässig schaltbar sind. Wird nicht nur eine Steuerung des Drucks in den Betätigungselementen 12 benötigt, sondern auch eine Regelung, und müssen die Steuerventile 33 hierfür mit hoher Frequenz sehr schnell und zuverlässig schaltbar sein, so bietet sich dafür eine Konstruktion aus gestapelten Piezoelementen an. Neben derartigen Ventilen und klassischen Magnetventilen können auch Ventile eingesetzt werden, die mit Piezomotoren betätigt werden.

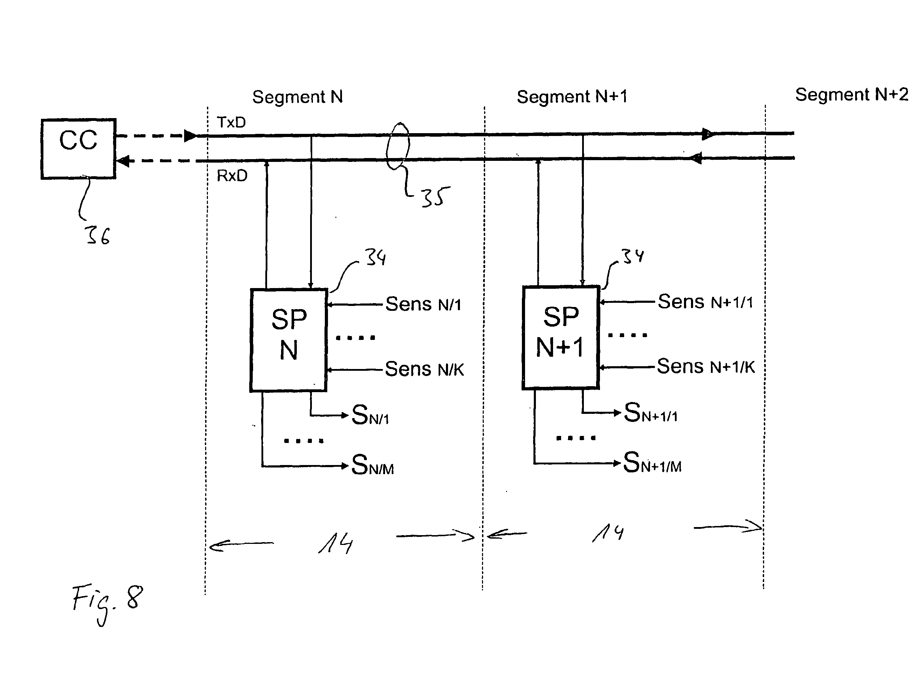

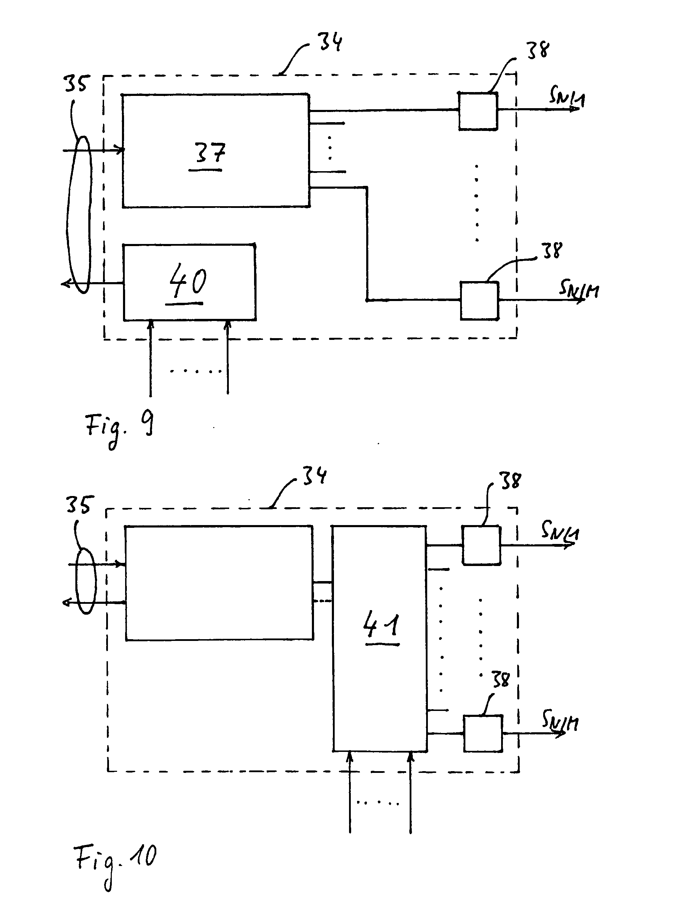

Wie in Figur 3 und 8 dargestellt ist, weist jede Betätigungseinheit 14 eine Treiberschaltung 34 auf, die über einen Bus 35 mit einer zentralen Steuerschaltung 36 verbunden ist. Die Treiberschaltung 34, die Schalt- oder Steuersignale S_{N/1}, ... S_{N/M} für die Ventile 33 der Betätigungseinheit 14 in Abhängigkeit von der zentralen Steuerschaltung 36 empfangenen Steuerinformation liefert, kann eine Steuerinformationsverarbeitungsschaltung 37 umfassen (siehe Figur 9), der von der zentralen Steuerschaltung 36 die Stelldaten für die entsprechenden Ventile 33 zum Einstellen des in jedem der Betätigungselemente 12 benötigten Arbeitsdruck mitgeteilt wird.

Es ist aber auch denkbar, dass die zentrale Steuerschaltung 36 nur Information oder Daten bezüglich der von dem Mechanismus auszuführenden Bewegung oder der einzunehmenden Form an die Steuerinformationsverarbeitungsschaltungen 37 der Treiberschaltungen 34 der einzelnen Betätigungseinheiten 14 liefert, während der Steuerinformationsverarbeitungskreis 37 in jeder der Betätigungseinheiten 14 aus den empfangenen Daten die entsprechenden einzustellenden Sollwerte für die einzelnen Betätigungselemente 12 selbst lokal ermittelt und so die Betätigungselemente 12 über die Ventile 33 mit entsprechendem Arbeitsmedium beaufschlagt. Zum Schalten der Ventile 33 weist die Treiberschaltung 34 vorzugsweise für jedes Ventil 33 einen entsprechenden Treiberkreis 38 auf, der die logischen Steuersignale in Betätigungssignale umsetzt.

Um nicht nur eine Steuerung der Relativbewegung der beiden Stützplatten 11 einer Betätigungseinheit 14 gegeneinander zu erhalten, sondern auch eine Regelung der eingestellten Positionen durchführen zu können, sind vorteilhafterweise in jeder Betätigungseinheit 14 ein oder mehrere Bewegungsfühler 39 vorgesehen, deren Ausgangssignale an die Treiberschaltung 34 geliefert werden.

Für den Fall, dass der Treiberschaltung 34 von der zentralen Steuerschaltung 36 die für die jeweiligen Betätigungselemente 12 erforderlichen Stelldaten übertragen werden, wenn also diese Daten aus der durchzuführenden Bewegung oder einzunehmenden Position zentral berechnet werden, können die Ausgangssignale der Bewegungsfühler 39 von der Treiberschaltung 34 über einen Übertragungskreis 40 und den Bus 35 an die zentrale Steuerschaltung 36 zurückübertragen werden, die dann die Stelldaten für die einzelnen Betätigungselemente 12 für eine Regelung nachberechnet.

Wird gemäß einer anderen Ausgestaltung der Erfindung mit einer verteilten Intelligenz oder Rechenkapazität gearbeitet und werden nur Information oder Daten über die auszuführende Bewegung oder einzunehmende Stellung an die einzelnen Treiberkreise 34 der Betätigungseinheiten 14 geliefert, und ermitteln dort die Steuerinformationsverarbeitungsschaltungen 37 selbst lokal die entsprechenden Stelldaten für die einzelnen Betätigungselemente 14, die von den Ventiltreibern 38 in entsprechende Steuersignale S_{N/1}, ... S_{N/M} umgesetzt werden, so ist zweckmäßigerweise in jeder Treiberschaltung 34 ein Reglerkreis 41 vorgesehen, der aus den Sollwerten der Stellwerte und den über die Bewegungsfühler 39 erfassten Ist-Werten der erreichten Verformung einen geänderten Stellwert für die Nachregelung ermittelt.

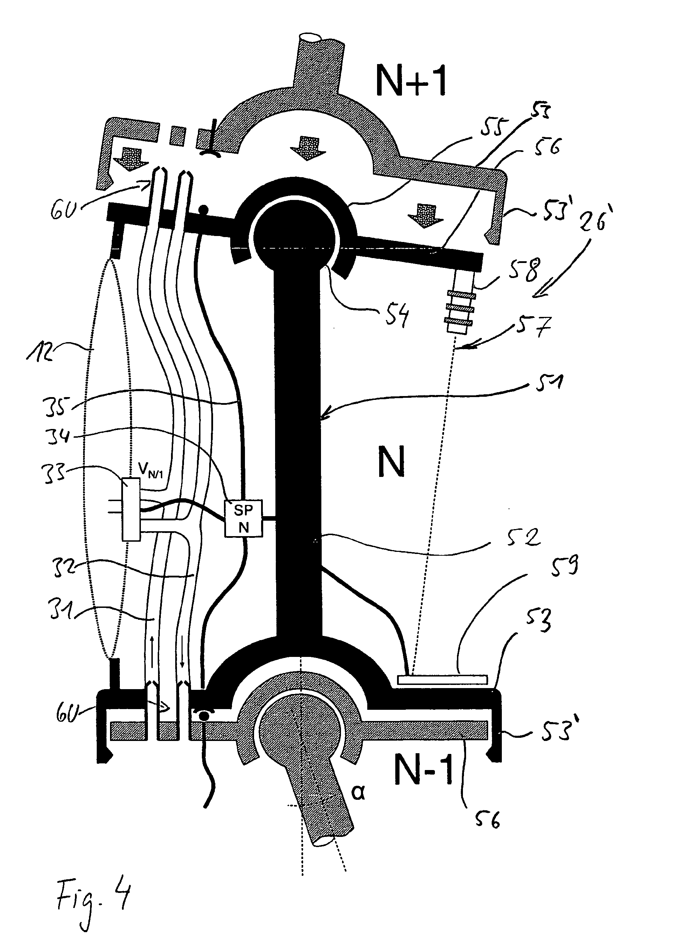

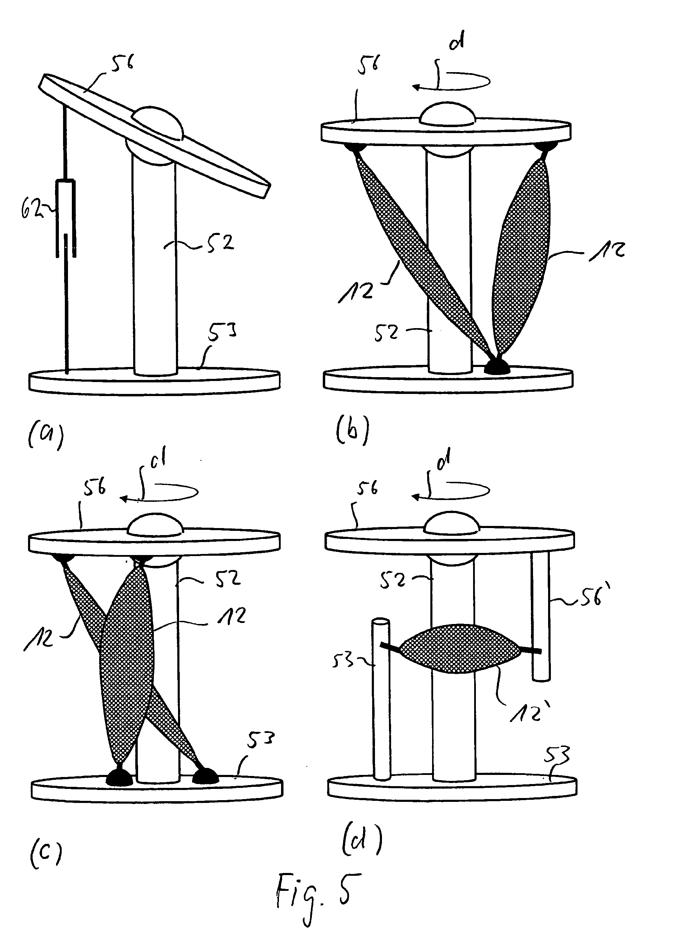

Als Bewegungsfühler zur Erfassung der Relativbewegung der Stützplatten 11 einer Betätigungseinheit 14 gegeneinander und der Krümmung der Trägerstruktur 10 in den einzelnen Betätigungseinheiten 14 können als Bewegungsfühler auf der Trägerstruktur Sensoren, beispielsweise Dehnungsmessstreifen vorgesehen werden. Anstelle von Dehnungsmessstreifen können aber auch Winkelencoder, geeignet angeordnete Tauchspulen oder optische Lagedetektoren (siehe Figur 4) vorgesehen sein. Eine Tauchspule 62 ist beispielsweise in Figur 5(a) angedeutet. Mit Hilfe derartiger Sensoren lässt sich also eine sehr genaue Regelung des wurmförmigen Mechanismus erzielen, wobei es vorteilhaft ist, dass die Ventile 33, insbesondere bei der Verwendung gestapelter Piezoelemente, als Ventilantrieb sehr schnell schaltbar sind, so dass die für einen zu erzielenden Bewegungsschritt durchgelassene Menge an Arbeitsmedium sehr klein sein kann, was einen entsprechenden kleinen Stellschritt zur Folge hat und damit eine hohe Einstellgenauigkeit liefert.

Bei dem in den Figuren 1 und 3 dargestellten wurmförmigen Mechanismus erfolgt die Zuleitung des Arbeitsmediums durch die kontinuierlich verformbare Trägerstruktur 10. Bei wurmförmigen Arbeitsmechanismen, die mit einem Arbeitsmedium arbeiten, das in der entsprechenden Umgebung ohne weiteres freigesetzt werden kann, kann dabei auch vorgesehen sein, dass die Trägerstruktur 10 selbst nicht nur als Stütz- oder Wellschlauch, sondern auch als Druckschlauch zur Zuführung des Arbeitsmediums zu den einzelnen Ventilen 33 dient.

Neben der durch die Trägerstruktur 10 geführten zentralen Leitung 31, 32 für das Arbeitsmedium kann die zentrale Leitung 31. 32 auch außerhalb der Trägerstruktur 10 geführt sein. In Figur 4 ist dies bei einem Betätigungselement 14 eines wurmförmigen Mechanismus mit wirbelsäulenartiger Trägerstruktur 21 gezeigt, der modulartig aufgebaut ist.

Ähnlich wie in Figur 2 dargestellt, weist die Trägerstruktur der Betätigungseinheit 26' in Figur 4 ein Trägersegment 51 mit einem Längsträger 52 und einer daran angeordneten Stützplatte 53 auf. Der Längsträger 52 weist an seinem von der Stützplatte 53 abgewandten Ende ein Gelenkglied 54 auf, auf das ein Gelenklager 55 aufgesetzt ist, das eine zweite Stützplatte 56 trägt. Das von dem Gelenkglied 54 und dem Gelenklager 55 gebildete Gelenk kann je nach den gewünschten Bewegungsfreiheitsgraden eine Drehbewegung um die Längsachse des Längsträgers 52 und/oder eine Schwenk- oder Kippbewegung relativ dazu in ein oder zwei Freiheitsgraden ermöglichen.

Die erste Stützplatte 53 weist auf ihrer vom Längsträger 52 abgewandten Seite als Axialflansch 53' angedeutete Befestigungsmittel zur Halterung einer zweiten Stützplatte 56 einer anschließenden oder vorhergehenden Betätigungseinheit 26' auf.

In der ersten und zweiten Stützplatte sind als Stecker oder Kupplungen angedeutete erste und zweite Verbindungsmittel 60 vorgesehen, um die einzelnen Abschnitte des Busses 35, der Arbeitsmedium-Zuführleitung 31 und der Rücklauf- oder Ablassleitung 32 miteinander zu verbinden, so dass sich der Bus 35 und die Leitungen 31, 32 kontinuierlich durch den modulartig aufgebauten, wurmförmigen Mechanismus hindurch erstrecken. Bei einer wirbelsäulenartigen Trägerstruktur, wie sie in Figur 2 gezeigt ist, können die Leitungen 31, 32 und gegebenenfalls auch der Bus 35 wie bei der Ausführungsform nach Figur 3 kontinuierlich durch den Mechanismus geführt sein.

Als Bewegungsfühler ist in Figur 4 ein optischer Detektor 57 dargestellt, der eine Lichtquelle, vorzugsweise einen Laser 58, insbesondere eine Laserdiode und einen positionsempfindlichen Fotodetektor 59 umfasst. Laser 58 und positionsempfindlicher Detektor 59 sind dabei an einander gegenüber liegenden Seiten der ersten und zweiten Stützplatten 53, 56 angeordnet.

Obwohl in Figur 4 nur ein Betätigungselement 12 dargestellt ist, das über ein Ventil 33 mit der Zuführleitung 31 und der Rücklauf- oder Ablassleitung 32 verbindbar ist, können je nach Anforderungen zwei, drei oder mehr Betätigungselemente 12 vorgesehen sein.

Anstelle einer Verbiegung der Trägerstruktur 10 oder einer Verkippung der beiden Stützelemente 11 einer Betätigungseinheit 14 relativ zueinander, wie dies anhand der Figuren 1 bis 4 und 5(a) dargestellt ist, kann durch eine entsprechende Anordnung der Betätigungselemente 12 und der Auslegung von Gelenkglied 24 und Gelenklager 25 erreicht werden, dass die Stützplatten 56, 53 nur eine Drehbewegung gegeneinander ausführen. Hierzu sind zunächst Gelenkglied 24 und Gelenklager 25 so ausgebildet, dass sie nur eine Drehbewegung der oberen Stützplatte 56 in Figur 5(a) bis (d) gegenüber dem Längsträger 52 des Trägersegments 51 zulässt. Wie in Figur 5(b) dargestellt, sind die beiden Betätigungselemente 12, die als künstliche Muskeln angedeutet sind, V-förmig angeordnet, so dass die Kontraktion des in der Zeichnung rechten Betätigungselements 12 eine Verdrehung der oberen Stützplatte 56 gegenüber der unteren in Richtung des Pfeils d ausgeführt hat.

Wird nun das rechte Betätigungselement 12 entlastet und gleichzeitig das linke Betätigungselement 12 mit Druck beaufschlagt, so kann das erstere gestreckt werden, während sich das letztere zusammenzieht, um die obere Stützplatte 56 entgegnen dem Pfeil d zurückzubewegen.

In entsprechender Weise arbeiten die beiden Betätigungselemente 12, die in Figur 5(c) in X-förmiger Anordnung gezeigt sind.

Anstelle der zwischen den beiden Stützplatten 56, 53 vorgesehenen Betätigungselemente 12 ist es auch möglich, die Stützplatten 56, 53 mit Lagerelementen 53', 56' auszurüsten, die als Haltestäbe dargestellt sind und ein Betätigungselement 12' dazwischen aufzuspannen.

Je nach Anwendungszweck können Drehbewegungen und Schwenk- oder Kippbewegungen getrennt von einzelnen Betätigungseinheiten 14 übernommen werden. Es ist aber ebenso möglich, Dreh- und Schwenk-/Kippbewegungen in einer Betätigungseinheit 14 zu kombinieren.

Wird in einer Betätigungseinheit 14 ein oder mehrere Betätigungselemente 12 eingesetzt, die sich gleichförmig ausdehnen oder zusammenziehen, so lässt sich auch eine Verlängerung oder Verkürzung der Betätigungseinheit 14 erzielen.

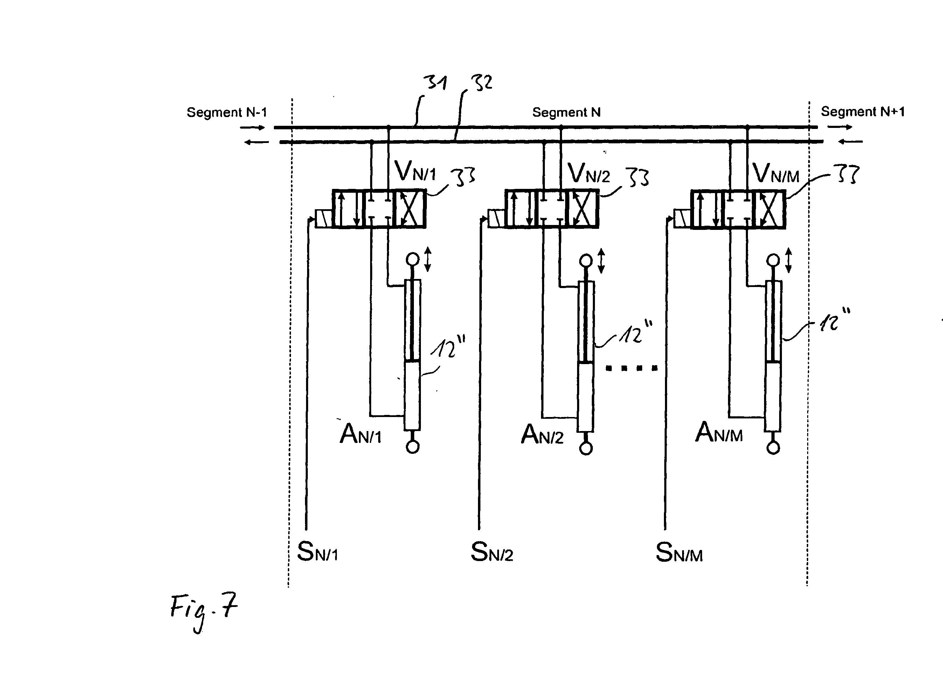

Anstelle der bisher beschriebenen Betätigungselemente 12, die als sich unter Druck zusammenziehende pneumatische oder hydraulische Arbeitselemente, insbesondere als so genannte künstliche Muskeln beschrieben wurden, ist es auch möglich, als Betätigungselemente Druckzylinder 12" vorzusehen, wie dies in Figur 7 dargestellt ist. Derartige Druckzylinder 12", die je nach Verbindung ihrer Arbeitskammern mit den Zu- und Abführleitungen 31, 32 in der einen oder anderen Richtung bewegbar sind, lassen sich die entsprechenden Stützelemente 11 in beiden Arbeitsrichtungen aktiv gegeneinander verstellen.

Werden nur in einer Richtung aktiv arbeitende Betätigungselemente 12, wie eingangs beschrieben, verwendet, so ist es zweckmäßig, dass die Betätigungselemente 12 bei Druckentlastung eine Rückstellwirkung liefern. Es ist aber auch möglich, dass jedem Betätigungselement 12 einer Betätigungseinheit 14 ein oder mehrere entgegengesetzt wirkende Betätigungselemente 12 zugeordnet sind, wie dies beispielsweise bei der anhand von Figur 3 beschriebenen Betätigungseinheit 14 der Fall ist.

Statt die Betätigungselemente 12 selbst mit einer bei Druckentlastung wirkenden Rückstellwirkung auszustatten, ist es auch möglich, den einzelnen Betätigungselementen 12 ein oder mehrere separate Rückstellelemente 61 zuzuordnen, wie in Figur 1 in den beiden rechten Betätigungseinheiten 14 angedeutet ist.

Sind in einer oder mehreren der Betätigungseinheiten des erfindungsgemäßen wurmförmigen Mechanismus, wie in Figur 2 auf der rechten Seite angedeutet. Zug- und/oder Druckfederelemente 63 vorgesehen, die zum Ausgleich von in einer geplanten Anwendungslage des Mechanismus zu erwartenden statischen Kräften diesen intern abstützen, so können diese Federelemente gegebenenfalls auch gleichzeitig als Rückstellelemente für die Betätigungselemente 12 dienen. Derartige Zug- oder Druckfederelemente lassen sich dabei auch als eine außen um den wurmförmigen Mechanismus herum angeordnete umhüllende Federanordnung ausbilden.

## Patentansprüche

1. Wurmförmiger Mechanismus mit:
- einer sich in Längsrichtung des Mechanismus erstreckenden Trägerstruktur (10), und
- zumindest zwei in Längsrichtung der Trägerstruktur (10) hintereinander angeordneten Betätigungseinheiten (14), wobei
-- jeder Betätigungseinheit (14) zwei Stützelemente (11) zugeordnet sind, die sich quer zur Längsrichtung der Trägerstruktur (10) erstreckend an dieser in Längsrichtung von einander beabstandet angebracht sind, und
-- jede Betätigungseinheit (14) zumindest ein zwischen den Stützelementen (11) angeordnetes, hydraulisches oder pneumatisches Betätigungselement (12) aufweist, das an eine zentrale Zuführleitung (31) zum Zuführen eines hydraulischen oder pneumatischen Arbeitsmediums angeschlossen ist und durch das die Stützelemente (11) relativ zu einander bewegbar sind, und wobei
jedes der Betätigungselemente (12) an die Zuführleitung (31) angeschlossen ist, **darduch gekennzeichnet,** dass
- dieser Anschluss über ein Ventil (33) erfolgt, welches über eine zentrale Leitung (35) einzeln oder in Gruppen ansteuerbar ist, und
- dass als Betätigungselemente (12) Druckzylinder vorgesehen sind, die je nach Verbindung ihrer Arbeitskammern mit Zu- und Abführleitungen in beiden Richtungen bewegbar sind.

2. Wurmförmiger Mechanismus mit:
- einer sich in Längsrichtung des Mechanismus erstreckenden Trägerstruktur (10), und
- zumindest zwei in Längsrichtung der Trägerstruktur (10) hintereinander angeordneten Betätigungseinheiten (14), wobei
-- jeder Betätigungseinheit (14) zwei Stützelemente (11) zugeordnet sind, die sich quer zur Längsrichtung der Trägerstruktur (10) erstreckend an dieser in Längsrichtung von einander beabstandet angebracht sind, und
-- jede Betätigungseinheit (14) zumindest ein zwischen den Stützelementen (11) angeordnetes, hydraulisches oder pneumatisches Betätigungselement (12) aufweist, das an eine zentrale Zuführleitung (31) zum Zuführen eines hydraulischen oder pneumatischen Arbeitsmediums angeschlossen ist und durch das die Stützelemente (11) relativ zu einander bewegbar sind, und wobei
der Betätigungselemente (12) an die Zuführleitung (31) angeschlossen ist, **darduch gekennzeichnet,** dass
- dieser Anschluss über ein Ventil (33) erfolgt, welches über eine zentrale Leitung (35) einzeln oder in Gruppen ansteuerbar ist, und
- dass als Betätigungselemente (12) sich unter Druck zusammenziehende pneumatische oder hydraulische Arbeitselemente, insbesondere so genannte künstliche Muskeln vorgesehen sind.

3. Mechanismus nach Anspruch 1, **dadurch gekennzeichnet, dass** die Ventile (33) jeweils eine erste offene Stellung zum Verbinden des Betätigungselements (12) mit der Zuführleitung (31), eine Schließstellung und eine zweite offene Stellung zum Auslassen des Arbeitsmediums aus dem Betätigungselement (12) aufweisen, wobei die Ventile (33) in der zweiten offenen Stellung die Betätigungselemente (12) mit einer Rücklaufleitung oder mit einer Auslassleitung (32) verbinden.

4. Mechanismus nach Anspruch 3, **dadurch gekennzeichnet, dass** die Ventile (33) mittels einer Konstruktion gestapelter Piezoelemente oder mittels Piezomotoren schaltbar sind.

5. Mechanismus nach einem der Ansprüche 1 bis 4, **dadurch gekennzeichnet, dass** jede Betätigungseinheit (14) eine Treiberschaltung (34) aufweist, die über die zentrale Leitung (35) mit einer zentralen Steuerschaltung (36) verbunden ist und die Schaltsignale für die Ventile (33) der Betätigungseinheit (14) in Abhängigkeit von von der zentralen Steuerschaltung (36) empfangenen Steuerinformation liefert.

6. Mechanismus nach Anspruch 5, **dadurch gekennzeichnet, dass** die Treiberschaltung (34) einen Steuerinformationsverarbeitungskreis (37) umfasst, der Steuersignale für die Ventile (33) schaltende Ventiltreiberkreise (38) in Abhängigkeit von der empfangenen Steuerinformation erzeugt.

7. Mechanismus nach Anspruch 5, oder 6, **dadurch gekennzeichnet, dass** jede Betätigungseinheit (14) einen oder mehrere Bewegungsfühler (39) aufweist, deren Ausgangssignale von der Treiberschaltung (34) erfasst werden.

8. Mechanismus nach Anspruch 7, **dadurch gekennzeichnet, dass** die Treiberschaltung (34) einen Übertragungskreis (40) für die Übertragung der Ausgangssignale von dem oder den Bewegungsfühlern (39) an die zentrale Steuerschaltung (36) aufweist, oder dass die Treiberschaltung (34) einen Reglerkreis (41) aufweist, dem die von der zentralen Steuerschaltung (36) empfangene Steuerinformation und die Ausgangssignale von dem oder den Bewegungsfühlern (39) als Regelinformation zugeführt sind und der Steuersignale für die die Ventile (33) schaltende Ventiltreiberkreise (38) in Abhängigkeit von der Regelinformation liefert.

9. Mechanismus nach Anspruch 7 oder 8, **dadurch gekennzeichnet, dass** als Bewegungsfühler (39) ein oder mehrere Dehnungsmessstreifen vorgesehen sind, die auf der Trägerstruktur (10) angebracht sind, oder dass als Bewegungsfühler (39) eine oder mehr Tauchspulen (62) vorgesehen sind, die so zwischen den Stützelementen (11) der Betätigungseinheit (14) angeordnet sind, dass sie deren Relativbewegung erfassen, oder dass als Bewegungsfühler (39) ein optischer Lagedetektor mit einer Lichtquelle und einem positionsempfindlichen Photoempfänger vorgesehen ist, die jeweils an gegeneinander bewegbaren Elementen der Betätigungseinheit (14) angeordnet sind, wobei der Lagedetektor ein der relativen Bewegung zwischen diesen Elementen entsprechendes Ausgangssignal liefert.

10. Mechanismus nach Anspruch 2, **dadurch gekennzeichnet, dass** die Betätigungselemente (12) bei Druckentlastung eine Rückstellwirkung liefern, wobei jedem Betätigungselement (12) einer Betätigungseinheit (14) ein oder mehrere entgegengesetzt wirkende Betätigungselemente (12) zugeordnet sind.

11. Mechanismus nach Anspruch 2, **dadurch gekennzeichnet, dass** den Betätigungselementen (12) ein oder mehrere Rückstellelemente (61) zugeordnet sind.

12. Mechanismus nach einem der vorstehenden Ansprüche, **dadurch gekennzeichnet, dass** das oder die Betätigungselemente (12) in der Betätigungseinheit (14) so angeordnet sind, dass sie bei Aktivierung eine Längenänderung der Betätigungseinheit (14) bewirken.

13. Mechanismus nach einem der Ansprüche 1 bis 12, **dadurch gekennzeichnet, dass** in einer Betätigungseinheit (14) zwei, drei oder mehr Betätigungselemente (12) so angeordnet sind, dass sie bei entsprechender Aktivierung eine definierte Verkippung der beiden Stützelemente (11) gegeneinander in einer definierten radialen Richtung bewirken, oder dass die Betätigungselemente (12) so in einer Betätigungseinheit (14) angeordnet sind, dass sie bei Aktivierung eine Verdrehung der beiden Stützelemente (11) um die Längsrichtung der Trägerstruktur (10) bewirken.

14. Mechanismus nach einem der vorstehenden Ansprüche, **dadurch gekennzeichnet, dass** die Stützelemente (11) als Stützscheiben ausgebildet sind, die einen Durchgang für die zentrale Leitung (31, 35) aufweisen.

15. Mechanismus nach einem der vorstehenden Ansprüche, **dadurch gekennzeichnet, dass** die Trägerstruktur (10) einen kontinuierlich verformbaren rohr- oder schlauchförmigen Träger aufweist, an dem die Stützelemente (11) in definierten axialen Abständen befestigt sind.

16. Mechanismus nach einem der Ansprüche 1 bis 15, **dadurch gekennzeichnet, dass** die Trägerstruktur (10) aus einer Mehrzahl von gleichartigen Trägersegmenten (21, 51) besteht, die modulartig miteinander verbunden sind.

17. Mechanismus nach Anspruche 16, **dadurch gekennzeichnet, dass** jede der Betätigungseinheiten (14) ein Trägersegment (51) aufweist, das eine erste Stützplatte (53), die fest mit einem Ende eines Längsträgers (22) verbunden ist, und eine zweite Stützplatte (56) umfasst, die über eine Gelenkverbindung (54, 55) schwenkbar und/oder drehbar an dem anderen Ende des Längsträgers (52) gehalten ist, wobei die erste Stützplatte (53) auf ihrer vom Längsträger (52) abgewandten Seite Befestigungsmittel (53') zum Befestigen einer zweiten Stützplatte (53) eines benachbarten Trägersegments aufweist, oder dass jede der Betätigungseinheiten (14) ein Trägersegment (21) umfasst, das eine Stützplatte (23) aufweist, die fest mit einem Ende eines Längsträgers (22) verbunden ist und die auf ihrer vom Längsträger (22) abgewandten Seite ein Gelenklager (25) trägt, wobei der Längsträger (22) an seinem anderen Ende ein mit dem Gelenklager (25) korrespondierendes Gelenkglied (24) trägt, so dass der Längsträger (22) einer Betätigungseinheit (14) schwenkbar und/oder drehbar in dem Gelenklager (25) an der Stützplatte (23) der nächsten Betätigungseinheit (14) lagerbar ist.

18. Mechanismus nach einem der vorstehenden Ansprüche, **dadurch gekennzeichnet, dass** in einem oder mehreren der Betätigungseinheiten (14) Zugfeder- und/oder Druckfederelemente vorgesehen sind, die zum Ausgleich von in einer geplanten Anwendungslage des Mechanismus zu erwartenden statischen Kräften diesen intern abstützen.

19. Modulartige Betätigungseinheit für einen wurmförmiger Mechanismus mit
- ersten und zweiten Stützplatten (53, 56), die sich quer zur Längsrichtung eines Längsträgers (52) erstrecken, wobei
-- die erste Stützplatte (53) fest mit einem Ende des Längsträgers (52) verbunden ist,
-- die zweite Stützplatte (56) über eine Gelenkverbindung (54, 55) schwenkbar und/oder drehbar an dem anderen Ende des Längsträgers (52) gehalten ist und
-- die eine Stützplatte (53) auf seiner vom Längsträger (52) abgewandten Seite Befestigungsmittel (53') zum Befestigen einer zweiten Stützplatte (56) einer benachbarten Betätigungseinheit aufweist, und
- zumindest einem zwischen den Stützplatten (53, 56) angeordnetem, hydraulischen oder pneumatischen Betätigungselementen (12), das an eine zentrale Zuführleitung (31) des wurmförmigen Mechanismus zum Zuführen eines hydraulischen oder pneumatischen Arbeitsmediums anschließbar ist und durch das die Stützplatten (53, 56) relativ zu einander bewegbar sind,
- wobei jedes der Betätigungselemente (12) über ein Ventil (33) an die Zuführleitung (31) anschließbar ist, das über eine zentrale Leitung (35) des wurmförmigen Mechanismus ansteuerbar ist, und
- wobei
- als Betätigungselemente (12) Druckzylinder vorgesehen sind, die je nach Verbindung ihrer Arbeitskammern mit Zu- und Abführleitungen in beiden Richtungen bewegbar sind oder
- als Betätigungselemente (12) sich unter Druck zusammenziehende pneumatische oder hydraulische Arbeitselemente, insbesondere so genannte künstliche Muskeln vorgesehen sind.

## Claims

1. A worm-like mechanism having:
- a support structure (10) extending in the longitudinal direction of the mechanism, and
- at least two actuating units (14) arranged one behind another in the longitudinal direction of the support structure (10), wherein
-- two supporting elements (11) are assigned to each actuating unit (14), the supporting elements (11) are fitted mutually spaced apart in the longitudinal direction on the support structure (10) in a fashion transverse to the longitudinal direction of the latter, and
-- each actuating unit (14) has at least one hydraulic or pneumatic actuating element (12) that is arranged between the supporting elements (11), is connected to a central feed line (31) for feeding a hydraulic or pneumatic working medium and by means of which the supporting elements (11) can be moved relative to one another, and
- wherein each of the actuating elements (12) is connected to the feed line (31), **characterized in that**
- this connection is carried out via a valve (33) that can be activated individually or in groups via a central line (35), and
- pressure cylinders are provided as actuating elements (12), the pressure cylinders can be moved in both directions depending on the connection of their working chambers with feed and discharge lines.

2. A worm-like mechanism having:
- a support structure (10) extending in the longitudinal direction of the mechanism, and
- at least two actuating units (14) arranged one behind another in the longitudinal direction of the support structure (10), wherein
-- two supporting elements (11) are assigned to each actuating unit (14), the supporting elements (11) are fitted mutually spaced apart in the longitudinal direction on the support structure (10) in a fashion transverse to the longitudinal direction of the latter, and
-- each actuating unit (14) has at least one hydraulic or pneumatic actuating element (12) that is arranged between the supporting elements (11), is connected to a central feed line (31) for feeding a hydraulic or pneumatic working medium and by means of which the supporting elements (11) can be moved relative to one another, and
- wherein each of the actuating elements (12) is connected to the feed line (31), **characterized in that**
- this connection is carried out via a valve (33) that can be activated individually or in groups via a central line (35), and
- provided as actuating elements (12) are pneumatic or hydraulic working elements contracting under pressure, in particular so-called artificial muscles.

3. The mechanism as claimed in claim 1, **characterized in that** the valves (33) in each case has a first open position for connecting the actuating element (12) to the feed line (31), a closed position and a second open position for letting the working medium out of the actuating element (12), wherein in the second open position the valves (33) connect the actuating elements (12) to a return line or to an outlet line (32).

4. The mechanism as claimed in claim 3, **characterized in that** the valves (33) can be switched by means of a structure of stacked piezoelements or by means of piezomotors.

5. The mechanism as claimed in one of claims 1 to 4, **characterized in that** each actuating unit (14) has a driver circuit (34) that is connected to a central control circuit (36) via the central line (35) and that supplies switching signals for the valves (33) of the actuating unit (14) as a function of control information received from the central control circuit (36).

6. The mechanism as claimed in claim 5, **characterized in that** the driver circuit (34) comprises an open-loop control information processing circuit (37) that generates control signals for valve driver circuits (38) switching the valves (33), as a function of the received open-loop control information.

7. The mechanism as claimed in claim 5 or 6, **characterized in that** each actuating unit (14) has one or more movement sensors (39) whose output signals are detected by the driver circuit (34).

8. The mechanism as claimed in claim 7, **characterized in that** the driver circuit (34) has a transmission circuit (40) for transmitting the output signals from the movement sensor(s) (39) to the central control circuit (36), or **in that** the driver circuit (34) has a closed-loop controller circuit (41) to which, as closed-loop control information, the open-loop control information received from the central control circuit (36) and the output signals from the movement sensor(s) (39) are fed, and that supplies open-loop control signals for the valve driver circuits (38) switching the valves (33) as a function of the closed-loop control information.

9. The mechanism as claimed in claim 7 or 8, **characterized in that** provided as movement sensors (39) are one or more strain gauges that are fitted on the support structure (10), or **in that** provided as movement sensors (39) are one or more plunger coils (62) that are arranged between the supporting elements (11) of the actuating unit (14) such that they detect the relative movement thereof, or **in that** provided as movement sensor (39) is an optical position detector with a light source and a position-sensitive photoreceiver that are respectively arranged on elements of the actuating unit (14) that can be moved toward one another, the position detector supplying an output signal corresponding to the relative movement between these elements.

10. The mechanism as claimed in claim 2, **characterized in that** the actuating elements (12) supply a restoring effect upon pressure relief, wherein one or more oppositely acting actuating elements (12) are assigned to each actuating element of an actuating unit (14).

11. The mechanism as claimed in claim 2, **characterized in that** one or more restoring elements (61) are assigned to the actuating elements (12).

12. The mechanism as claimed in one of the preceding claims, **characterized in that** the actuating element(s) (12) is/are arranged in the actuating unit (14) such that upon activation they effect a change in the length of the actuating unit (14).

13. The mechanism as claimed in one of claims 1 to 12, **characterized in that** two, three or more actuating elements (12) are arranged in an actuating unit (14) such that upon appropriate activation they effect a defined tilting of the two supporting elements (11) toward one another in a defined radial direction, or **in that** the actuating elements (12) are arranged in an actuating unit (14) such that upon activation they effect a rotation of the two supporting elements (11) about the longitudinal direction of the support structure (10).

14. The mechanism as claimed in one of the preceding claims, **characterized in that** the supporting elements (11) are designed as supporting disks that have a passage for the central line (31,35).

15. The mechanism as claimed in one of the preceding claims, **characterized in that** the support structure (10) has a continuously deformable tubular- or hose-like carrier on which the supporting elements (11) are fastened at defined axial spacings.

16. The mechanism as claimed in one of claims 1 to 15, **characterized in that** the support structure (10) consists of a plurality of similar support segments (21, 51) that are modularly interconnected.

17. The mechanism as claimed in claim 16, **characterized in that** each of the actuating units (14) has a support segment (51) that comprises a first supporting disk (53) that is permanently connected to one end of a longitudinal carrier (22), and a second supporting disk (56) that is held pivotably and/or rotatably on the other end of the longitudinal carrier (52) via an articulated connection (54, 55), the first supporting disk (53) having, on its side averted from the longitudinal carrier (52), fastening means (53') for fastening a second supporting disk (53) of an adjacent support segment, or **in that** each of the actuating units (14) comprises a support segment (21) that has a supporting disk (23) that is permanently connected to one end of a longitudinal carrier (22) and that carries an articulated bearing (25) on its side averted from the longitudinal carrier (22), the longitudinal carrier (22) carrying on its other end an articulated member (24) corresponding to the articulated bearing (25) such that the longitudinal carrier (22) of an actuating unit (14) can be pivotably and/or rotatably supported in the articulated bearing (25) on the supporting disk (23) of the next actuating unit (14).

18. The mechanism as claimed in one of the preceding claims, **characterized in that** provided in one or more of the actuating units (14) are tension spring and/or compression spring elements that support the mechanism internally in order to balance static forces which are to be expected in a planned position of use of said mechanism.

19. An modular actuating unit for a worm-like mechanism having
- first and second supporting disks (53, 56) that extend transverse to the longitudinal direction of a longitudinal carrier (52), wherein
-- the first supporting disk (53) is permanently connected to one end of the longitudinal carrier (52),
-- the second supporting disk (56) is held pivotably and/or rotatably on the other end of the longitudinal carrier (52) via an articulated connection (54, 55), und
-- the first supporting disk (53) having, on its side averted from the longitudinal carrier (52), fastening means (53') for fastening a second supporting disk (56) of an adjacent support segment, and
- at least one hydraulic or pneumatic actuating element (12) that is arranged between the supporting disks (53, 56) can be connected to a central feed line (31) of the worm-like mechanism for feeding a hydraulic or pneumatic working medium and by means of which the supporting disks (53, 56) can be moved relative to one another,
- wherein each of the actuating elements (12) can be connected to the feed line (31) via a valve (33) that can be activated via a central line (35) of the worm-like mechanism, and
- wherein pressure cylinders are provided as actuating elements (12), the pressure cylinders can be moved in both directions depending on the connection of their working chambers with feed and discharge lines (31, 32) or wherein provided as actuating elements (12) are pneumatic or hydraulic working elements contracting under pressure, in particular so-called artificial muscles.

## Revendications

1. Mécanisme vermiforme comprenant :
- une structure porteuse (10) s'étendant en direction longitudinale du mécanisme, et
- au moins deux unités actionnement (14) agencées les unes derrière les autres en direction longitudinale de la structure porteuse (10), dans lequel
-- à chaque unité d'actionnement (14) sont associés deux éléments de soutien (11), qui sont montés sur la structure porteuse (10) à distance l'un de l'autre en direction longitudinale et qui s'étendent transversalement à la direction longitudinale de la structure porteuse (10), et
-- chaque unité d'actionnement (14) comprend au moins un élément d'actionnement (12) hydraulique ou pneumatique agencé entre les éléments de soutien (11), qui est raccordé à une conduite d'alimentation centrale (31) pour l'alimentation d'un fluide de travail hydraulique ou pneumatique et au moyen desquels les éléments de soutien (11) sont déplaçables les uns par rapport aux autres, et dans lequel
- chacun des éléments d'actionnement (12) est raccordé à la conduite d'alimentation (31),
**caractérisé en ce que**
ce raccordement a lieu via une valve (33) qui est susceptible d'être pilotée soit individuellement soit par groupe via une conduite centrale (35), et
- **en ce qu'**il est prévu à titre d'éléments d'actionnement (12) des cylindres sous pression qui, selon la liaison de leurs chambres de travail avec des conduites d'amenée et d'évacuation, sont déplaçables dans les deux directions.

2. Mécanisme vermiforme comprenant :
- une structure porteuse (10) s'étendant en direction longitudinale du mécanisme, et
- au moins deux unités actionnement (14) agencées les unes derrière les autres en direction longitudinale de la structure porteuse (10), dans lequel
-- à chaque unité d'actionnement (14) sont associés deux éléments de soutien (11), qui sont montés sur la structure porteuse (10) à distance l'un de l'autre en direction longitudinale et qui s'étendent transversalement à la direction longitudinale de la structure porteuse (10), et
-- chaque unité d'actionnement (14) comprend au moins un élément d'actionnement (12) hydraulique ou pneumatique agencé entre les éléments de soutien (11), qui est raccordé à une conduite d'alimentation centrale (31) pour l'alimentation d'un fluide de travail hydraulique ou pneumatique et au moyen desquels les éléments de soutien (11) sont déplaçables les uns par rapport aux autres, et dans lequel
- chacun des éléments d'actionnement (12) est raccordé à la conduite d'alimentation (31),
**caractérisé en ce que**
ce raccordement a lieu via une valve (33) qui est susceptible d'être pilotée soit individuellement soit par groupe via une conduite centrale (35), et
- **en ce qu'**il est prévu à titre d'éléments d'actionnement (12) des éléments de travail pneumatiques ou hydrauliques qui se contractent sous pression, en particulier ce que l'on appelle des muscles artificiels.

3. Mécanisme selon la revendication 1, **caractérisé en ce que** les valves (33) présentent respectivement une première position ouverte pour relier l'élément d'actionnement (12) avec la conduite d'alimentation (31), une position fermée et une seconde position ouverte pour laisser sortir le fluide de travail hors de l'élément d'actionnement (12), dans lequel, dans la seconde position ouverte, les valves (33) relient les éléments d'actionnement (12) avec une conduite de retour ou avec une conduite d'échappement (32).

4. Mécanisme selon la revendication 3, **caractérisé en ce que** les valves (33) sont susceptibles d'être commutées au moyen d'une construction d'éléments piézoélectriques empilés ou au moyen de moteurs piézoélectriques.

5. Mécanisme selon l'une des revendications 1 à 4, **caractérisé en ce que** chaque unité d'actionnement (14) comprend un circuit pilote (34), qui est relié à un circuit de commande central (36) via la conduite centrale (35) et fournit les signaux de commutation pour les valves (33) de l'unité d'actionnement (14) en fonction d'informations de commande reçues par le circuit de commande central (36).

6. Mécanisme selon la revendication 5, **caractérisé en ce que** le circuit pilote (34) comprend un circuit de traitement d'informations de commande (37), qui engendre des signaux de commande pour des circuits de pilotage de valve (38) qui font commuter les valves en fonction des informations de commande reçues.

7. Mécanisme selon la revendication 5 ou 6, **caractérisé en ce que** chaque unité d'actionnement (14) comprend un ou plusieurs capteurs de déplacement (39), dont les signaux de sortie sont détectés par le circuit de pilotage (34)

8. Mécanisme selon la revendication 7, **caractérisé en ce que** le circuit pilote (34) comprend un circuit de transmission (40) pour la transmission des signaux de sortie depuis le ou les capteur(s) de déplacement (39) vers le circuit de commande central (36), ou **en ce que** le circuit pilote (34) comprend un circuit de régulation (41) auquel sont admis, à titre d'informations de régulation, les informations de commande reçues par le circuit de commande centrale (36) et les signaux de sortie depuis le ou les capteur(s) de déplacement (39), et qui délivre les signaux de commande pour les circuits de pilotage de valve (38) qui font commuter les valves (33), en fonction des informations de régulation.

9. Mécanisme selon la revendication 7 8, **caractérisé en ce que** l'on prévoit un titre de capteur de déplacement (39) une ou plusieurs jauges de mesure d'allongement, qui sont montées sur la structure porteuse (10), **en ce que** l'on prévoit à titre de capteur de déplacement (39) une ou plusieurs bobines mobiles (62) qui sont agencées entre les éléments de soutien (11) de l'unité d'actionnement (14) de telle façon qu'elles détectent leur mouvement relatif, ou bien **en ce que** l'on prévoit à titre de capteur de déplacement (39) un détecteur optique de position avec une source de lumière et un récepteur photoélectrique sensible en position, qui sont agencés respectivement sur des éléments, mobiles les uns par rapport aux autres, de l'unité d'actionnement (14), et le détecteur de position délivre un signal de sortie correspondant au mouvement relatif entre ces éléments.

10. Mécanisme selon la revendication 2, **caractérisé en ce que** les éléments d'actionnement (12) délivrent, lors de la décharge de pression, un effet de rappel, et un ou plusieurs éléments d'actionnement (12) à effet opposé est/sont associé(s) à chaque élément d'actionnement (12) d'une unité d'actionnement (14).

11. Mécanisme selon la revendication 2, **caractérisé en ce qu'**un ou plusieurs éléments de rappel (61) est/sont associé(s) aux éléments d'actionnement (12).

12. Mécanisme selon l'une des revendications précédentes, **caractérisé en ce que** le ou les élément(s) d'actionnement (12) dans l'unité d'actionnement (14) est/sont agencé(s) de telle façon qu'ils entraînent, lors d'une activation, une modification de longueur de l'unité d'actionnement (14).

13. Mécanisme selon l'une des revendications 1 à 12, **caractérisé en ce que**, dans une unité d'actionnement (14), deux, trois ou plusieurs éléments d'actionnement (12) sont agencés de telle façon que lors d'une activation correspondante, ils provoquent un basculement défini des deux éléments de soutien (11) l'un par rapport à l'autre dans une direction radiale définie, ou **en ce que** les éléments d'actionnement (12) sont agencés dans une unité d'actionnement (14) de telle façon que, lors d'une activation, ils provoquent une rotation des deux éléments de soutien (11) autour de la direction longitudinale de la structure porteuse (10).

14. Mécanisme selon l'une des revendications précédentes, **caractérisé en ce que** les éléments de soutien (11) sont réalisés sous forme de disques de soutien qui présentent une traversée pour la conduite centrale (31, 35).

15. Mécanisme selon l'une des revendications précédentes, **caractérisé en ce que** la structure porteuse (10) comprend un support en forme de tube ou de tuyau déformable de manière continue, sur lequel sont fixés les éléments de soutien (11) à des distances axiales définies.

16. Mécanisme selon l'une des revendications 1 à 15, **caractérisé en ce que** la structure porteuse (10) est composée d'une pluralité de segments porteurs (21, 51) de même type, qui sont reliés les uns aux autres à la manière de modules.

17. Mécanisme selon la revendication 16, **caractérisé en ce que** chacune des unités d'actionnement (14) comprend un segment porteur (51), qui inclut une première plaque de soutien (53) qui est reliée fermement à une extrémité d'un support longitudinal (22), et une seconde plaque de soutien (56) qui est maintenue via une liaison articulée (54, 55) en basculement et/ou en pivotement à l'autre extrémité du support longitudinal (52), et la première plaque de soutien (53) comprend, sur son côté détourné du support longitudinal (52), des moyens de fixation (53') pour la fixation d'une seconde plaque de soutien (53) d'un segment porteur voisin, ou **en ce que** chacune des unités d'actionnement (14) inclut un segment porteur (21) qui comprend une plaque de soutien (23) qui est reliée fermement avec une extrémité d'un support longitudinal (22) et qui porte un palier d'articulation (25) sur son côté détourné du support longitudinal (22), et le support longitudinal (22) porte à son autre extrémité un élément d'articulation (24) correspondant au palier d'articulation (25), de sorte que le support longitudinal (22) d'une unité d'actionnement (14) peut être montée avec possibilité de basculement et/ou de rotation dans le palier d'articulation (25) sur la plaque de soutien (23) de l'unité d'actionnement suivante (14).

18. Mécanisme selon l'une des revendications précédentes, **caractérisé en ce que**, dans une ou plusieurs des unités d'actionnement (14) il est prévu des éléments de ressort en traction et/ou des éléments de ressort en compression qui soutiennent de façon interne ce mécanisme afin de compenser les forces statiques à attendre dans une situation d'application prévue du mécanisme.

19. Unité d'actionnement modulaire d'un mécanisme vermiforme, comprenant
- une première et une seconde plaque de soutien (53, que 56), qui s'étendent transversalement à la direction longitudinale d'un support longitudinal (52), dans laquelle
-- la première plaque de soutien (53) est fermement reliée avec une extrémité du support longitudinal (52),
-- la seconde plaque de soutien (56) est maintenue via une liaison articulée (54, 55) avec possibilité de basculement et/ou de rotation sur l'autre extrémité du support longitudinal (52), et
-- la première plaque de soutien (53) comprend, sur son côté détourné du support longitudinal (52), des moyens de fixation (53') pour la fixation d'une seconde plaque de soutien (56) d'une unité d'actionnement voisine, et
- au moins un élément d'actionnement (12) hydraulique ou pneumatique agencé entre les plaques de soutien (53, 56), qui peut être raccordé à une conduite d'alimentation centrale (31) du mécanisme vermiforme pour l'admission d'un fluide de travail hydraulique ou pneumatique, et au moyen desquels les plaques de soutien (53, 56) sont déplaçable l'une par rapport à l'autre,
- dans laquelle chacun des éléments d'actionnement (12) est susceptible d'être raccordé via une valve (33) à la conduite d'alimentation (31), laquelle peut être pilotée via une conduite centrale (35) du mécanisme vermiforme, et
dans laquelle
- il est prévu à titre d'éléments d'actionnement (12) des cylindres sous pression qui, selon la liaison de leurs chambres de travail avec des conduites d'amenée et d'évacuation, sont déplaçables dans les deux directions, ou
- il est prévu à titre d'éléments d'actionnement (12) des éléments de travail pneumatiques ou hydrauliques qui se contractent sous pression, en particulier ce que l'on appelle des muscles artificiels.
